(19) Europäisches Patentamt
European Patent Office
Office européen des brevets

(11) Veröffentlichungsnummer : **0 060 423**
**B1**

# (12) EUROPÄISCHE PATENTSCHRIFT

(45) Veröffentlichungstag der Patentschrift : **11.04.84**

(51) Int. Cl.³ : **C 07 D213/64**, A 01 N 43/40

(21) Anmeldenummer : **82101510.4**

(22) Anmeldetag : **27.02.82**

## (54) 2-Pyridyloxy-acetanilide, Verfahren zu ihrer Herstellung und ihre Verwendung als Herbizide.

(30) Priorität : **12.03.81 JP 34555/81**

(43) Veröffentlichungstag der Anmeldung : **22.09.82 Patentblatt 82/38**

(45) Bekanntmachung des Hinweises auf die Patenterteilung : **11.04.84 Patentblatt 84/15**

(84) Benannte Vertragsstaaten : **AT BE CH DE FR GB IT LI NL**

(56) Entgegenhaltungen :
**CH-A- 625 940**
**DE-A- 2 637 886**
**GB-A- 1 472 485**
**US-A- 4 110 104**

(73) Patentinhaber : **NIHON TOKUSHU NOYAKU SEIZO K.K.**
**No.4, 2-chome, Nihonbashi Honcho Chuo-ku**
**Tokyo 103 (JP)**

(72) Erfinder : **Aya, Masahiro**
**2-844, Suzuki-cho**
**Kodaira-shi Tokyo (JP)**
Erfinder : **Saito, Junichi**
**3-7-12, Osawa**
**Mitaka-shi Tokyo (JP)**
Erfinder : **Yasui, Kazuomi**
**7-6-15, Shakujii-dai**
**Nerima-ku Tokyo (JP)**
Erfinder : **Kagabu, Shinzo**
**47-15, Oya-cho**
**Hachioji-shi Tokyo (JP)**
Erfinder : **Kamochi, Atsumi**
**2-24-10, Higashi-Toyoda**
**Hino-shi Tokyo (JP)**
Erfinder : **Yamaguchi, Naoko**
**3064-1, Hino**
**Hino-shi Tokyo (JP)**

(74) Vertreter : **Gremm, Joachim, Dr. et al**
**Bayer AG c/o Zentralbereich Patente, Marken und Lizenzen**
**D-5090 Leverkusen 1, Bayerwerk (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 060 423 B1

2-Pyridyloxy-acetanilide, Verfahren zu ihrer Herstellung und ihre Verwendung als Herbizide

Die vorliegende Erfindung betrifft bestimmte 2-Pyridyloxy-acetanilide, ein Verfahren zu deren Herstellung sowie deren Verwendung als Herbizide.

Die GB-PS 1 472 485 offenbart, daß 6-Fluoro-3,5-dihalogeno-2-pyridyloxamid-Verbindungen der allgemeinen Formel

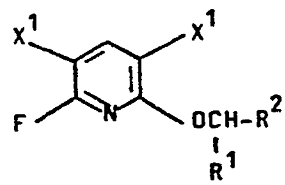

(VI)

in der

$X^1$ für Chlor, Brom oder Iod,

$R^1$ für Wasserstoff oder Methyl und

$R^2$ für $—CONR^4R^4$,

worin $R^4$ Wasserstoff oder ein Alkyl mit 1 bis 8 Kohlenstoff-Atomen bezeichnet, stehen, herbizide Wirksamkeit besitzen.

Gegenstand der vorliegenden Erfindung sind nunmehr, als Verbindungen, 2-Pyridyl-oxyacetanilide der allgemeinen Formel

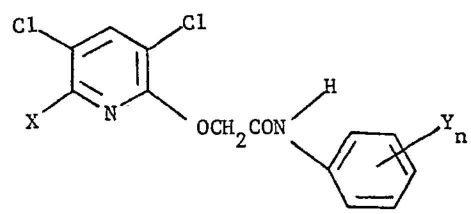

(I)

in der

X Wasserstoff, $C_1$-$C_4$-Alkyl (d. h. Methyl, Ethyl, n-oder iso-Propyl, oder n-, iso-, sec- oder tert-Butyl) oder $C_1$-$C_4$-Halogenoalkyl bezeichnet,

Y Halogen (d. h. Fluor, Chlor, Brom oder Iod), $C_1$-$C_4$-Alkyl, $C_1$-$C_4$-Alkoxy (d. h. Methoxy, Ethoxy, n- oder iso-Propoxy oder n-, iso-, sec-, oder tert-Butoxy) oder $C_1$-$C_4$-Halogenoalkyl bezeichnet und

n 0, 1, 2 oder 3 ist, wobei die Substituenten Y unabhängig voneinander ausgewählt werden, wenn n 2 oder 3 ist.

Gegenstand der vorliegenden Erfindung ist außerdem ein Verfahren zur Herstellung einer Verbindung der Formel (I), bei dem eine 2-Pyridyl-oxyessigsäure der allgemeinen Formel

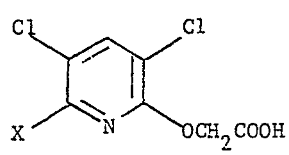

(II)

in der

X die im Vorstehenden angegebene Bedeutung hat, und ein Anilin der allgemeinen Formel

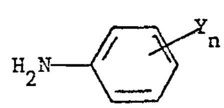

(III)

in der

Y und n die im Vorstehenden angegebenen Bedeutungen haben, unter Wasserabspaltung miteinander kondensiert werden.

Es wurde gefunden, daß die Verbindungen der Formel (I) hervorragende herbizide Wirksamkeit sowie ein breites Wirkungsspektrum besitzen. Außerdem besitzen sie eine sehr niedrige Toxizität gegenüber warmblütigen Tieren.

Die Verbindungen gemäß der vorliegenden Erfindung haben in Tests eine stärkere herbizide Wirkung bewiesen als gewisse nach dem Stand der Technik offenbarte Verbindungen. Damit repräsentiert die

vorliegende Erfindung einen erheblichen technischen Fortschritt.

Bevorzugte Verbindungen der Formel (I) sind solche, in denen

X Wasserstoff, Methyl, Mono-, Di- oder Trichloromethyl oder Mono-, Di- oder Trifluoromethyl bezeichnet,

Y Fluor, Chlor, Brom, Methyl, Ethyl, n-Propyl, Isopropyl, Methoxy, Ethoxy, n-Propoxy, Isopropoxy, Mono-, Di- oder Trichloromethyl oder Mono-, Di- oder Trifluoromethyl bezeichnet und

n 0, 1 oder 2 ist.

Beispiele für die Verbindungen der vorliegenden Erfindung sind : 3,5-Dichloro-2-pyridyloxyacetanilid, 3,5-Dichloro-2-pyridyloxyaceto-2- (oder 3- oder 4-) -chloroanilid, 3,5-Dichloro-6-methyl-2-pyridyloxyacetanilid, 3,5-Dichloro-2-pyridyloxyaceto-2- (oder 3- oder 4-)-bromoanilid, 3,5-Dichloro-2-pyridyloxyaceto-2- (oder 3- oder 4-)-fluoroanilid, 3,5-Dichloro-2-pyridyloxyaceto-2- (oder 3- oder 4-)-methylanilid, 3,5-Dichloro-2-pyridyloxyaceto-2- (oder 3- oder 4-)-ethylanilid, 3,5-Dichloro-2-pyridyloxyaceto-2- (oder 3- oder 4-)-propylanilid, 3,5-Dichloro-2-pyridyloxyaceto-2- (oder 3- oder 4-)-isopropylanilid, 3,5-Dichloro-2-pyridyloxyaceto-2- (oder 3- oder 4-)-methoxyanilid, 3,5-Dichloro-2-pyridyloxyaceto-2- (oder 3- oder 4-)-ethoxyanilid, 3,5-Dichloro-2-pyridyloxyaceto-2- (oder 3- oder 4-)-isopropoxyanilid, 3,5-Dichloro-2-pyridyloxyaceto-2- (oder 3- oder 4-)-trifluoromethylanilid, 3,5-Dichloro-2-pyridyloxyaceto-2,4- (oder 2,6)-dichloroanilid, 3, 5-Dichloro-2-pyridyloxyaceto-2,4,5- (oder 2,4,6)-trichloroanilid, 3,5-Dichloro-2-pyridyloxyaceto-2,3-dimethylanilid, 3,5-Dichloro-2-pyridyloxyaceto-2,4-dimethylanilid, 3,5-Dichloro-6-trifluoromethyl-2-pyridyloxyacetanilid und 3,5-Dichloro-6-trichloromethyl-2-pyridyloxyacetanilid.

Wenn 3,5-Dichloro-6-methyl-2-pyridyloxyessigsäure und Anilin als die Ausgangsstoffe bei dem Verfahren gemäß der vorliegenden Erfindung verwendet werden, kann der Reaktionsablauf durch die nachstehende Gleichung dargestellt werden :

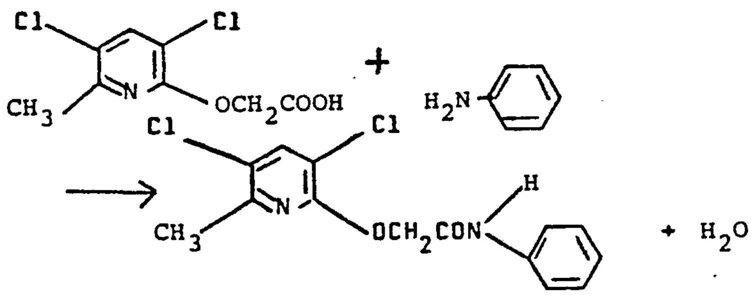

Beispiele für die 2-Pyridyloxyessigsäure der Formel (II), die als Ausgangsstoffe bei dem Verfahren der vorliegenden Erfindung verwendet können, sind :

3,5-Dichloro-2-pyridyloxyessigsäure, 3,5-Dichloro-6-methyl-2-pyridyloxyessigsäure, 3,5-Dichloro-6-ethyl-2-pyridyloxyessigsäure, 3,5-Dichloro-6-n- (oder iso-) propyl-2-pyridyloxyessigsäure, 3,5-Dichloro-6-n- (oder iso-, sec- oder tert-) butyl-2-pyridyloxyessigsäure, 3,5-Dichloro-6-chloromethyl- (oder dichloromethyl- oder trichloromethyl-)-2-pyridyloxyessigsäure oder 3,5-Dichloro-6-fluoromethyl- (oder difluoromethyl- oder trifluoromethyl-)-2-pyridyloxyessigsäure.

Beispiele für das Anilin der Formel (III), die ebenfalls als Ausgangsstoffe bei dem Verfahren der vorliegenden Erfindung verwendet werden können, sind :

Anilin, 2- (oder 3- oder 4-) Chloroanilin, 2- (oder 3- oder 4-) Bromoanilin, 2- (oder 3- oder 4-) Fluoroanilin, 2- (oder 3- oder 4-) Methylanilin, 2- (oder 3- oder 4-)-Ethylanilin, 2- (oder 3- oder 4-) Propyl- (oder Isopropyl-) anilin, 2- (oder 3- oder 4-)-n-Butyl- (oder iso-, sec- oder tert-Butyl-) anilin, 2- (oder 3- oder 4-) Methoxyanilin, 2- (oder 3- oder 4-) Ethoxyanilin, 2- (oder 3- oder 4-) Propoxy- (oder Isopropoxy-) anilin, 2- (oder 3- oder 4-)-n-Butoxy- (oder iso-, sec- oder tert-Butoxy-)-anilin, 2-(oder 3- oder 4-) Chloromethyl- (oder Dichloromethyl- oder Trichloromethyl-) anilin, 2- (oder 3- oder 4-) Fluoromethyl- (oder Difluoromethyl- oder Trifluoromethyl-) anilin, 2,4- (oder 2,6-) Dichloroanilin, 2,3- (oder 2,4-) Dimethylanilin oder 2,4,5- (oder 2,4,6-) Trichloroanilin.

Das Verfahren gemäß der vorliegenden Erfindung wird vorzugsweise unter Verwendung eines Lösungsmittels oder Verdünnungsmittels durchgeführt. Zu diesem Zweck können sämtliche inerten Lösungsmittel oder Verdünnungsmittel verwendet werden.

Beispiele für solche Lösungsmittel oder Verdünnungsmittel sind Wasser ; aliphatische, alicyclische und aromatische (gegebenenfalls chlorierte) Kohlenwasserstoffe wie Hexan, Cyclohexan, Petrolether, Ligroin, Benzol, Toluol, Xylol, Methylenchlorid, Chloroform, Kohlenstofftetrachlorid, Ethylenchlorid, Trichloroethylen und Chlorbenzol ; Ether wie Diethylether, Methylethylether, Di-isopropylether, Dibutylether, Propylenoxid, Dioxan und Tetrahydrofuran ; Ketone wie Aceton, Methylethylketon, Methylisopropylketon und Methylisobutylketon ; Nitrile wie Acetonitril, Propionitril und Acrylnitril ; Alkohole wie Methanol, Ethanol, Isopropanol, Butanol und Ethylenglycol, Ester wie Ethylacetat und Amylacetat ; Säureamide wie Dimethylformamid und Dimethylacetamid ; Sulfone und Sulfoxide wie Dimethylsulfoxid und Sulfolan ; und Basen wie Pyridin.

In entsprechender Weise können auch Gemische aus Lösungsmitteln und/oder Verdünnungsmitteln verwendet werden.

Das erfindungsgemäße Verfahren beinhaltet eine unter Wasserabspaltung verlaufende Kondensationsreaktion. Diese Reaktion kann entweder unter Erhitzen des Reaktionsgemisches auf höhere Temperaturen, im allgemeinen auf Temperaturen zwischen 100 und 200 °C, oder in Gegenwart eines Kondensationsmittels bei Temperaturen zwischen – 20 °C und Raumtemperatur durchgeführt werden ; Beispiele für geeignete Kondensationsmittel sind Carbodiimide, Enamine und Tetrachlorsilikat. Es ist ferner möglich, das im Reaktionsverlauf gebildete Wasser durch azeotrope Destillation aus dem Reaktionsgemisch zu entfernen.

Die Wirkstoffe gemäß der vorliegenden Erfindung beeinflussen das Wachstum der Pflanzen und können dementsprechend als Entlaubungsmittel, Abbrandmittel, Mittel zur Zerstörung breitblättriger Pflanzen, keimungshemmende Mittel und, insbesondere, als Unkrautvernichtungsmittel verwendet werden. Unter Unkräutern im weitesten Sinne sind solche Pflanzen zu verstehen, die an Stellen wachsen, wo sie nicht erwünscht sind.

Ob die erfindungsgemäßen Verbindungen totale oder selektive Herbizid-Wirkung entfalten, hängt im wesentlichen von der angewandten Menge ab.

Die aktiven Verbindungen gemäß der vorliegenden Erfindung können beispielsweise zur Bekämpfung der folgenden Pflanzen verwendet werden :

Dikotyledonen-Unkräuter der Gattungen

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Rorippa, Rotala, Lindernia, Lamium, Veronica, Abutilon, Emex, Datura, Viola, Galeopsis, Papaver, Centaurea und Solanum ; und

Monokotyledonen-Unkräuter der Gattungen

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus und Apera.

Die aktiven Verbindungen gemäß der vorliegenden Erfindung können beispielsweise als selektive Herbizide in den folgenden Kulturen verwendet werden :

Dikotyledonen-Kulturen der Gattungen

Gossypium, Glycine, Beta, Daucus, Phaseolus, Pisum, Solanum, Linum, Ipomoea, Vicia Nicotiana, Lycopersicon, Arachis, Brassica, Lactuca, Cucumis und Cucurbita ; und

Monokotyledonen-Kulturen der Gattungen

Oryza, Zea, Triticum, Hordeum, Avena, Secale, Sorghum, Panicum, Saccharum, Ananas, Asparagus und Allium.

Die Anwendung der erfindungsgemäßen aktiven Verbindungen ist jedoch keineswegs auf diese Gattungen begrenzt, sondern umfaßt auch andere Pflanzen in gleicher Weise.

In Abhängigkeit von den Konzentrationen können die Verbindungen zur totalen Bekämpfung von Unkräutern, beispielsweise auf Industriegelände und Bahnkörpern sowie auf Wegen und Plätzen mit oder ohne Baumbestand, verwendet werden. Ebenso können die Verbindungen auch zur Unkrautbekämpfung in perennierenden Kulturen, beispielsweise Aufforstungen, Zierbaumpflanzungen, Obstgärten, Weinbergen und -gärten, Zitrushainen, Nußbaumpflanzungen, Bananenplantagen, Kaffeeplantagen, Teeplantagen, Gummiplantagen, Ölpalmenplantagen, Kakaoplantagen, Weichfruchtplantagen und Hopfenfeldern, sowie zur selektiven Unkrautbekämpfung in Jahreskulturen verwendet werden.

Die vorliegenden Verbindungen sind sehr wirksam, wenn sie zur Bekämpfung der in Reisfeldern auftretenden Unkräuter angewandt werden, und zeigen keine merkliche Phytotoxizität gegenüber den angebauten Reispflanzen. Die Verbindungen können vor, während oder nach dem Auflaufen der Unkräuter zur Anwendung gebracht werden. Sie können beispielsweise auf den Boden und/oder auf die Stengel und Blätter der Unkräuter aufgebracht werden. Als Beispiele für Reisfeld-Unkräuter seien hier erwähnt :

Rotala indica,
Lindernia procumbens,
Ludwiga prostrata,
Potamogeton distinctus,
Elatine triandra

sowie

Echinochloa crus-galli,
Monochoria vaginalis,
Eleocharis acicularis,
Eleocharis kuroguwai,
Cyperus difformis,
Cyperus serotinus,

Sagittaria pygmaea,
Alisma canaliculatum und
Scirpus juncoides.

Die Wirkstoffe können in übliche Präparate wie Lösungen, Emulsionen, benetzbare Pulver, Suspensionen, Stäubemittel, Pasten, lösliche Pulver, Granulat, Suspensions-Emulsions-Konzentrate, mit dem Wirkstoff imprägnierte natürliche und synthetische Materialien und sehr feine Kapseln in polymeren Substanzen, umgewandelt werden.

Diese Präparate können in bekannter Weise hergestellt werden, beispielsweise durch Vermischen der Wirkstoffe mit Streckmitteln, d. h. flüssigen oder aus verflüssigten Gasen bestehenden oder festen Verdünnungsmitteln oder Trägern, gegebenenfalls unter Verwendung von oberflächenaktiven Mitteln, d. h. Emulgatoren und/oder Dispergiermitteln und/oder schaumbildenden Mitteln. Bei Verwendung von Wasser als Streckmittel können beispielsweise auch organische Lösungsmittel als Hilfslösungsmittel verwendet werden.

Als flüssige Verdünnungsmittel oder Träger, insbesondere als Lösungsmittel, eignen sich hauptsächlich aromatische Kohlenwasserstoffe wie Xylol, Toluol oder Alkylnaphthaline, chlorierte aromatische oder chlorierte aliphatische Kohlenwasserstoffe wie Chlorbenzole, Chloroethylene oder Methylenchlorid, aliphatische oder alicyclische Kohlenwasserstoffe wie Cyclohexan oder Paraffine, beispielsweise Mineralöl-Fraktionen, Alkohole wie Butanol oder Glycol sowie deren Ether und Ester, Ketone wie Aceton, Methylethylketon. Methylisobutylketon oder Cyclohexanon, oder stark polare Lösungsmittel wie Dimethylformamid oder Dimethylsulfoxid, sowie auch Wasser.

Als feste Träger können gemahlene natürliche Minerale wie Kaoline, Tone, Talkum, Kreide, Quarz, Attapulgit, Montmorillonit oder Diatomeenerde, und gemahlene synthetische Minerale wie hochdisperse Kieselsäure, Aluminiumoxid und Silikate verwendet werden. Als feste Träger für Granulat kann zerkleinertes und fraktioniertes natürliches Gesteinsmaterial wie Calcit, Marmor, Bimsstein, Sepiolith und Dolomit sowie synthetisches Granulat aus anorganischen oder organischen Mehlen und Granulat aus organischem Material wie Sägemehl, Kokosnußschalen, Maiskolben und Tabakstengeln verwendet werden.

Als Emulgatoren und/oder Schaumbildner können nichtionische oder anionische Emulgatoren wie Polyoxyethylen-Fettsäureester, Polyoxyethylen-Fettalkoholether, beispielsweise Alkylarylpolyglycol ether, Alkylsulfonate, Alkylsulfate, und Arylsulfonate sowie Hydrolyseprodukte des Albumins verwendet werden. Zu den Dispergiermitteln zählen beispielsweise Ligninsulfit-Ablaugen und Methylcellulose.

Haftmittel wie Carboxymethylcellulose und natürliche und synthetische Polymerisate in Form von Pulver, Granulat oder Latices, z. B. Gummiarabicum, Polyvinylalkohol und Polyvinylacetat, können in den Formulierungen verwendet werden.

Es ist möglich, farbgebende Stoffe wie anorganische Pigmente, beispielsweise Eisenoxid, Titanoxid und Preußischblau, und organische Farbstoffe wie Alizarin-Farbstoffe, Azo-Farbstoffe und Metallphthalocyanin-Farbstoffe, und Spurennährstoffe wie Salze von Eisen, Mangan, Bor, Kupfer, Cobalt, Molybdän und Zink, zu verwenden.

Die Präparate enthalten im allgemeinen von 0,01 bis 100 Gewichts-%, vorzugsweise von 0,05 bis 95 Gewichts-% des Wirkstoffes.

Die Wirkstoffe gemäß der vorliegenden Erfindung können als solche oder in Form ihrer Präparate zur Unkrautbekämpfung auch im Gemisch mit bekannten Herbiziden verwendet werden, wobei gebrauchsfertige Formulierungen oder im Tankmischverfahren erhaltene Mittel einsetzbar sind. Auch Gemische mit anderen aktiven Verbindungen wie Fungiziden, Insektiziden, Akariziden, Nematiziden, vogelabweisenden Mitteln, Wachstumsfaktoren, Pflanzennährstoffen sowie Mitteln zur Verbesserung der Bodenstruktur sind einsetzbar.

Durch Einarbeiten anderer Wirkstoffe ist es möglich, ein breites herbizides Spektrum und eine gezielte Bekämpfungswirkung zu erreichen, und aufgrund der Vermischung mit solchen Wirkstoffen ist auch eine synergistische Wirkung zu erwarten. Beispiele für solche anderen Wirkstoffe sind :

Benzothiazol-2-yloxyaceto-N,N-diallylamid,
Benzoxazol-2-yloxyaceto-N-sec-butyl-N-methylamid,
Benzoxazol-2-yloxyaceto-N-cyclohexyl-N-methylamid,
Benzothiazol-2-yloxyaceto-N-methyl-N-(1-methyl-propargyl) amid,
Benzoxazol-2-yloxyaceto-N-benzyl-N-propargylamid,
Benzothiazol-2-yloxyaceto-2′-ethylpiperidid,
Benzothiazol-2-yloxyaceto-2′,4′-dimethylpiperidid,
Benzoxazol-2-yloxyaceto-2′,4′,6′-trimethylpiperidid,
Benzoxazol-2-yloxyaceto-hexamethylenimid,
Benzothiazol-2-yloxyaceto-perhydroindolid,
Benzoxazol-2-yloxyaceto-perhydroindolid,
Benzothiazol-2-yloxyaceto-1′2′,3′,4′-tetrahydrochinolid,
Benzoxazol-2-yloxyaceto-2′ methyl-1′2′,3′4′-tetrahydrochinolid,
Benzoxazol-2-yloxyaceto-N-methylanilid,

Benzothiazol-2-yloxyaceto-N-methylanilid,
Benzoxazol-2-yloxyaceto-N-ethylanilid,
Benzoxazol-2-yloxyaceto-N-isopropylanilid,
Benzoxazol-2-yloxyaceto-N-n-propylanilid,
Benzothiazol-2-yloxyaceto-N-methyl-N-2'-methoxyanilid,
Benzoxazol-2-yloxyaceto-N-methyl-N-2'-methoxyanilid,
Benzoxazol-2-yloxyaceto-N-methyl-N-2'-trifluoromethylanilid,
Benzothiazol-2-yloxyaceto-N-methyl-N-2'-chloroanilid,
Benzoxazol-2-yloxyaceto-N-methyl-N-2'-chloroanilid,
Benzothiazol-2-yloxyaceto-N-methyl-N-2'-fluoroanilid,
Benzoxazol-2-yloxyaceto-N-methyl-N-2'-fluoroanilid,
Benzothiazol-2-yloxyaceto-N-methyl-N-3'-methylanilid,
Benzoxazol-2-yloxyaceto-N-methyl-N-3'-methylanilid,
Benzothiazol-2-yloxyaceto-N-methyl-N-3'-methoxyanilid,
Benzoxazol-2-yloxyaceto-N-methyl-N-3'-methoxyanilid,
Benzothiazol-2-yloxyaceto-N-methyl-N-3'-isopropoxyanilid,
Benzothiazol-2-yloxyaceto-N-methyl-N-3'-trifluoromethylanilid,
Benzoxazol-2-yloxyaceto-N-methyl-N-3'-trifluoromethylanilid,
Benzothiazol-2-yloxyaceto-N-methyl-N-3'-chloroanilid,
Benzoxazol-2-yloxyaceto-N-methyl-N-3'-chloroanilid,
Benzothiazol-2-yloxyaceto-N-methyl-N-3'-fluoroanilid,
Benzoxazol-2-yloxyaceto-N-methyl-N-3'-fluoroanilid,
Benzothiazol-2-yloxyaceto-N-methyl-N-3'-bromoanilid,
Benzoxazol-2-yloxyaceto-N-methyl-N-3'-bromoanilid,
Benzoxazol-2-yloxyaceto-N-methyl-N-4'-methylanilid,
Benzoxazol-2-yloxyaceto-N-methyl-N-4'-methoxyanilid,
Benzoxazol-2-yloxyaceto-N-methyl-N-4'-fluoroanilid,
Benzoxazol-2-yloxyaceto-N-methyl-N-2',3'-dimethylanilid,
Benzoxazol-2-yloxyaceto-N-methyl-N-2',3'-dichloroanilid,
Benzoxazol-2-yloxyaceto-N-methyl-N-4'-chloro-2'-methylanilid,
Benzothiazol-2-yloxyaceto-N-methyl-N-2',5'-dichloroanilid,
Benzoxazol-2-yloxyaceto-N-methyl-N-2'5'-dichloroanilid,
Benzothiazol-2-yloxyaceto-N-methyl-N-3',5'-dimethylanilid,
Benzoxazol-2-yloxyaceto-N-methyl-N-3',5'-dimethylanilid,
Benzoxazol-2-yloxyaceto-N-methyl-N-3',5'-d-trifluoromethyl-anilid,
Benzoxazol-2-yloxyaceto-N-methyl-N-5'-indanylamid,
Benzothiazol-2-yloxyaceto-N-methyl-N-3'-ethylanilid,
Benzoxazol-2-yloxyaceto-N-methyl-N-3'-ethylanilid,
Benzothiazol-2-yloxyaceto-N-isopropylanilid,
2-Chloro-2',6'-diethyl-N-(butoxymethyl)-acetanilid,
2-Chloro-2',6'-diethyl-N-(n-propoxyethyl)-acetanilid,
N-(O,O-Dipropyl-diethyl-phosphorylacetyl)-2-methylpiperidin,
S-(4-Chlorobenzyl)-N,N-diethylthiolcarbamat,
S-Ethyl-N,N-hexamethylenthiolcarbamat,
O-Methyl-O-(2-nitro-p-tolyl)-N-isopropylphosphoroamidthioat,
O-Ethyl-O-(2-nitro-5-methylphenyl)-N-sec-butylphosphoroamidthioat,
Benzoxazol-2-yloxyaceto-N-methyl-N-3'-isopropoxyanilid,
3,4-Dimethyl-2,6-dinitro-N-1-ethylpropylanilid,
$\alpha,\alpha,\alpha$-Trifluoro-2,6-dinitro-N,N-dipropyl-p-toluidin,
4,5-Dichloro-1,3-thiazol-2-yloxyaceto-N-isopropyl-N-ethoxyethoxyamid, und
5-Ethyl-1,3,4-thiadiazol-2-yloxyaceto-1'2',3',4'-tetrahydrochinolid.

Die Wirkstoffe können als solche, in Form von Präparaten oder in Gebrauchsformen, die daraus durch weitere Verdünnung hergestellt werden, eingesetzt werden, beispielsweise in Form gebrauchsfertiger Lösungen, Emulsionen, Suspensionen, Pulver, Pasten und als Granulat.

Sie werden in üblicher Weise angewandt, beispielsweise durch Bewässern, Sprühen, Zerstäuben, Streuen oder Stäuben.

Die Mengen der aktiven Verbindung in den gebrauchsfertigen Präparaten können, den Umständen entsprechend, innerhalb eines breiten Rahmens variiert werden. Sie betragen im allgemeinen von 0,01 bis 95 Gewichts-%, vorzugsweise von 0,05 bis 60 Gewichts-%.

Die Wirkstoffe können nach dem Auflaufen der Pflanzen zur Anwendung gelangen, werden jedoch vorzugsweise vor dem Auflaufen der Pflanzen, d. h. mittels des Verfahrens der Vorauflaufbehandlung, angewandt. Sie können auch vor der Einsaat in den Boden eingearbeitet werden.

Die aufgewandten Wirkstoff-Mengen können innerhalb eines beträchtlichen Bereichs variiert werden. Im allgemeinen liegen die flächenbezogenen Aufwandsmengen des Wirkstoffs zwischen 0,1 und 3 kg/ha, vorzugsweise zwischen 0,2 und 1 kg/ha.

Die vorliegende Erfindung umfaßt weiter herbizide oder das Pflanzenwachstum regulierende Mittel, die als Wirkstoff eine Verbindung gemäß der vorliegenden Erfindung im Gemisch mit einem festen Verdünnungsmittel oder Träger oder im Gemisch mit einem ein oberflächenaktives Mittel enthaltenden flüssigen Verdünnungsmittel oder Träger enthalten.

Die vorliegende Erfindung umfaßt weiter ein Verfahren zur Unkrautbekämpfung, das dadurch gekennzeichnet ist, daß eine Verbindung gemäß der vorliegenden Erfindung allein oder in Form eines Präparats, das als Wirkstoff eine Verbindung gemäß der vorliegenden Erfindung im Gemisch mit einem Verdünnungsmittel oder Träger enthält, auf die Unkräuter oder deren Lebensraum aufgebracht wird.

Die vorliegende Erfindung umfaßt weiter ein Verfahren zur Regulierung des Pflanzenwachstums, das dadurch gekennzeichnet ist, daß eine Verbindung gemäß der vorliegenden Erfindung allein oder in Form eines Präparats, das als Wirkstoff eine Verbindung gemäß der vorliegenden Erfindung im Gemisch mit einem Verdünnungsmittel oder Träger enthält, auf die Pflanzen oder deren Lebensraum aufgebracht wird.

Die vorliegende Erfindung ist ferner auf Nutzpflanzen gerichtet, die dadurch gegen Schäden durch Unkräuter geschützt sind, daß sie auf Flächen angebaut werden, auf die unmittelbar vor und/oder während der Zeit des Anbauens eine Verbindung gemäß der vorliegenden Erfindung allein oder im Gemisch mit einem Verdünnungsmittel oder Träger aufgebracht wurde.

Die vorliegende Erfindung ist außerdem auf Pflanzen gerichtet, deren Wachstum dadurch reguliert wurde, daß sie auf Flächen angebaut wurden, auf die unmittelbar vor und/oder während des Anbauens eine Verbindung gemäß der vorliegenden Erfindung allein oder im Gemisch mit einem Verdünnungsmittel oder Träger aufgebracht wurde.

Es wird ersichtlich werden, daß die üblichen Methoden der Erzeugung geernteter Nutzpflanzen durch die vorliegende Erfindung verbessert werden können.

Die folgenden Beispiele dienen der weiteren Erläuterung der vorliegenden Erfindung.

## Beispiele

### Beispiel 1 — Herstellungsbeispiel

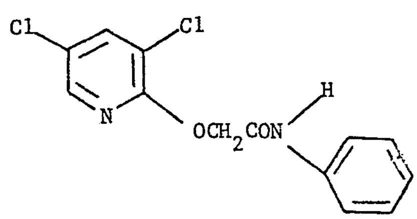

(1)

2,22 g 3,5-Dichloro-2-pyridyloxyessigsäure wurden in 30 ml Toluol suspendiert, und 2,06 g DCC* wurden hinzugefügt. Die Mischung wurde 15 min gerührt. Zu dieser Lösung wurden 0,93 g Anilin, gelöst in 5 ml Toluol, tropfenweise hinzugegeben. Die Reaktion war leicht exotherm, und das Rühren wurde 2 h bei Raumtemperatur fortgesetzt. Danach wurde die Reaktionsmischung filtriert. Nach dem Entfernen des Toluols aus dem Filtrat durch Destillation unter vermindertem Druck wurde der Rückstand mit einer kleinen Menge Ethanol gewaschen, wonach 2,52 g 3,5-Dichloro-2-pyridyloxyacetanilid (Ausbeute : 85 %) erhalten wurden ; Schmp. 164,5-166 °C.

Entsprechend der im Vorstehenden beschriebenen Verfahrensweise wurden die in Tabelle 1 aufgeführten Verbindungen erhalten.

Tabelle 1

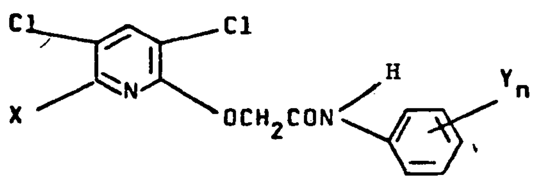

(I)

*DCC = N,N'-Dicyclohexylcarbodiimid (verwendet als Kondensationsmittel).

| Verbindung Nr. | X | $Y_n$ | Schmp. °C |
|---|---|---|---|
| 2 | $CH_3$ | H | 109,5- 110,5 |
| 3 | H | 2-F | 146 - 149 |
| 4 | H | 4-Cl | 153 - 154 |
| 5 | H | $2\text{-}CH_3$ | 164 - 165 |
| 6 | H | $3\text{-}CH_3$ | 140 - 141 |
| 7 | $CH_3$ | $4\text{-}CH_3$ | 137 - 138 |
| 8 | H | $4\text{-iso-}C_3H_7$ | 155 - 156 |
| 9 | H | $3\text{-}CF_3$ | 94 - 100 |
| 10 | H | $2\text{-}CH_3O\text{-}$ | 139 - 140 |
| 11 | $CH_3$ | $4\text{-}CH_3O\text{-}$ | 100 - 105 |
| 12 | H | $2\text{-iso-}C_3H_7O\text{-}$ | 127 - 129 |
| 13 | $CH_3$ | $2,5\text{-}Cl_2$ | 166 - 170 |
| 14 | H | $3,4\text{-}Cl_2$ | 168 - 169 |
| 15 | H | $2,3\text{-}(CH_3)_2$ | 175 - 176 |
| 16 | H | $2\text{-}CH_3\text{-}4\text{-}Cl$ | 166 - 167 |

Präparate gemäß der vorliegenden Erfindung werden in den folgenden Beispielen erläutert. Hierin werden die Verbindungen der vorliegenden Erfindung durch die (in Klammer angegebene) Zahl des betreffenden Hertellungsbeispiels bezeichnet.

Beispiel 2

15 Teile der Verbindung (1), 80 Teile eines 1 : 5-Gemisches aus pulvriger Diatomeenerde und Tonpulver, 2 Teile Natriumakylbenzolsulfonat und 3 Teile Natriumalkylnaphthalinsulfonat/Formaldehyd-Kondensat wurden pulverisiert und zu einem benetzbaren Pulver vermischt. Vor dem Gebrauch wurde das benetzbare Pulver mit Wasser verdünnt.

Beispiel 3

30 Teile der Verbindung (2), 55 Teile Xylol, 8 Teile Polyoxyethylen-alkylphenylether und 7 Teile Calciumalkylbenzolsulfonat wurden unter Rühren miteinander vermischt, wodurch ein emulgierbares Konzentrat erhalten wurde. Vor dem Gebrauch wurde das emulgierbare Konzentrat mit Wasser verdünnt.

Beispiel 4

2 Teile der Verbindung (3) und 98 Teile Tonpulver wurden pulverisiert und gemischt, wodurch ein Stäubemittel erhalten wurde.

Beispiel 5

1,5 Teile der Verbindung (4), 0,5 Teile Isopropylhydrogenphosphat (PAP) und 98 Teile Tonpulver wurden pulverisiert und gemischt, wodurch ein Stäubemittel erhalten wurde.

Beispiel 6

25 Teile Wasser wurden zu einer Mischung aus 10 Teilen der Verbindung (5), 30 Teilen Bentonit (Montmorillonit), 58 Teilen Talkum und 2 Teilen Ligninsulfonat-Salz zugesetzt, und das Gemisch wurde gut geknetet. Die Mischung wurde mittels eines Extruder-Granulators zu einem Granulat mit einer Korngröße von 0,43 bis 2,0 mm (10 bis 40 mesh) verarbeitet und bei 40 °C bis 50 °C getrocknet, wodurch ein Granulat erhalten wurde.

Beispiel 7

Ein Drehmischer wurde mit 95 Teilen Tonmineral-Teilchen mit einer Teilchengrößen-Verteilung im

Bereich von 0,2 bis 2 mm beschickt, und unter Drehen des Mischers wurden 6,5 Teile der Verbindung (6), gelöst in einem organischen Lösungsmittel, gleichmäßig auf die Tonmineral-Teilchen aufgesprüht. Die Teilchen wurden dann bei 40 °C bis 50 °C getrocknet, wodurch ein Granulat erhalten wurde.

Die herbizide Wirkung der Verbindungen wird durch die folgenden Beispiele aufgezeigt, in denen die Verbindungen der vorliegenden Erfindung durch die (in Klammer angegebene) Zahl gemäß dem vorstehenden Beispiel 1 und der Tabelle 1 bezeichnet werden.

Die bekannten Vergleichsverbindungen werden durch die nachstehenden Formeln beschrieben :

(VI – 1) =

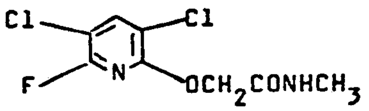

(VI – 2) =

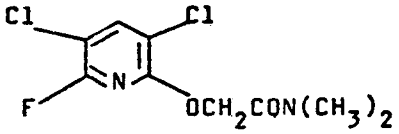

Diese Verbindungen wurden in der GB-PS 1 472 485 offenbart.

Beispiel 8

Prüfung der Wirkung gegen im Wasser wachsende Reisfeld-Unkräuter durch Behandlung des Bodens sowie der Stengel und Blätter unter Bewässerungs-Bedingungen (Topf-Test) :

Herstellung eines Wirkstoff-Präparates

Träger : 5 Gewichtsteile Aceton ;
Emulgator : 1 Gewichtsteil Benzyloxypolyglycolether.

Ein Wirkstoff-Präparat in Form eines emulgierbaren Konzentrats wurde durch Vermischen von 1 Teil der aktiven Verbindung mit den vorbezeichneten Mengen Träger und Emulgator erhalten. Eine vorbestimmte Menge des Präparats wurde durch Verdünnen mit Wasser erhalten.

Test-Verfahren

Wagner-Töpfe (2 $dm^2$) wurden mit Reis-Kulturboden gefüllt, und zwei Reis-Setzlinge (Varietät : Kinmaze) wurden in jeden Topf umgepflanzt. Samen von Echinochloa crus-galli, Cyperus iria, Monochoria vaginalis, Scirpus juncoides und bestimmten breitblättrigen Unkräutern, kleine Stücke von Eleocharis acicularis und Knollen von Cyperus serotinus und Sagittaria pygmaea wurden in die Töpfe gegeben, und die Töpfe wurden in feuchtem Zustand gehalten. Als Echinochloa crus-galli etwa bis zum 2-Blatt-Stadium gewachsen war (etwa 7 bis 9 Tage nach der Einsaat), wurden die Töpfe mit Wasser bis zu einer Tiefe von etwa 6 cm gefüllt, und eine vorher festgelegte Menge des Wirkstoffs wurde in Form einer Emulsion mittels einer Pipette zugegeben. Nach der Behandlung wurde das Wasser im Laufe von zwei Tagen mit einer Geschwindigkeit von 2 bis 3 cm pro Tag aus den Töpfen heraussickern gelassen. Danach wurde die Wassertiefe in den Töpfen bei etwa 3 cm gehalten, und vier Wochen nach der Behandlung mit dem Wirkstoff wurden die herbizide Wirkung und der Grad der Phytotoxizität mit Hilfe einer Skala von 0 bis 5 nach dem folgenden Maßstab bewertet.

Die Wirkungen wurden im Vergleich zu unbehandelten Kontrollpflanzen wie folgt bewertet :

| Bewertung | Unkrautvernichtungsrate (bezogen auf die unbehandelten Kontrollpflanzen) |
|---|---|
| 5 | mindestens 95 % (verdorrt) |
| 4 | mindestens 80 %, jedoch weniger als 95 % |
| 3 | mindestens 50 %, jedoch weniger als 80 % |
| 2 | mindestens 30 %, jedoch weniger als 50 % |
| 1 | mindestens 10 %, jedoch weniger als 30 % |
| 0 | weniger als 10 % (unwirksam) |

Die Phytotoxizität gegenüber den Reispflanzen wurde im Vergleich zu den unbehandelten Kontrollpflanzen wie folgt bewertet :

| Bewertung | Phytotoxizitätsrate (bezogen auf die unbehandelten Kontrollpflanzen) |
|---|---|
| 5 | mindestens 90 % (Ausrottung) |
| 4 | mindestens 50 %, jedoch weniger als 90 % |
| 3 | mindestens 30 %, jedoch weniger als 50 % |
| 2 | mindestens 10 %, jedoch weniger als 30 % |
| 1 | mehr als 0, jedoch weniger als 10 % |
| 0 | 0 % (keine Phytotoxizität) |

Die Testergebnisse sind in Tabelle 2 aufgeführt, in der die Symbole A bis H die Unkräuter wie folgt bezeichnen :

A : Echinochloa crus-galli Beauv. var.
B : Eleocharis acicularis L.
C : Cyperus iria L.
D : Scirpus juncoides Roxburgh var.
E : Monochoria vaginalis Presl
F : Breitblättrige Unkräuter (darunter Lindernia procumbens Philcox, Rotala indica Koehne, Elatine triandra Schkuhr)
G : Cyperus serotinus Rottboell
H : Sagittaria pygmaéa Miq.

Tabelle 2

| Verbindung | Wirkstoff-Menge kg/ha | Herbizide Wirkung A | B | C | D | E | F | G | H | Phytotoxität gegen Reis |
|---|---|---|---|---|---|---|---|---|---|---|
| (1) | 0,5 | 5 | 5 | 5 | 5 | 5 | 5 | 5 | 5 | 0 |
| (2) | 0,5 | 5 | 5 | 5 | 5 | 5 | 5 | 5 | 5 | 0 |
| (3) | 0,5 | 5 | 5 | 5 | 5 | 5 | 5 | 5 | 5 | 0 |
| (4) | 0,5 | 5 | 5 | 5 | 5 | 5 | 5 | 5 | 5 | 0 |
| (5) | 0,5 | 5 | 5 | 5 | 5 | 5 | 5 | 5 | 5 | 0 |
| (6) | 0,5 | 5 | 5 | 5 | 5 | 5 | 5 | 5 | 5 | 0 |
| (7) | 0,5 | 5 | 5 | 5 | 5 | 5 | 5 | 5 | 5 | 0 |
| (8) | 0,5 | 5 | 5 | 5 | 5 | 5 | 5 | 5 | 5 | 0 |
| (9) | 0,5 | 5 | 5 | 5 | 5 | 5 | 5 | 5 | 5 | 0 |
| (10) | 0,5 | 5 | 5 | 5 | 5 | 5 | 5 | 5 | 5 | 0 |
| (11) | 0,5 | 5 | 5 | 5 | 5 | 5 | 5 | 5 | 5 | 0 |
| (12) | 0,5 | 5 | 5 | 5 | 5 | 5 | 5 | 5 | 5 | 0 |
| (13) | 0,5 | 5 | 5 | 5 | 5 | 5 | 5 | 5 | 5 | 0 |
| (14) | 0,5 | 5 | 5 | 5 | 5 | 5 | 5 | 5 | 5 | 0 |
| (15) | 0,5 | 5 | 5 | 5 | 5 | 5 | 5 | 5 | 5 | 0 |
| (16) | 0,5 | 5 | 5 | 5 | 5 | 5 | 5 | 5 | 5 | 0 |
| (VI-1) | 0,5 | 2 | 4 | 4 | 2 | 3 | 4 | 2 | 3 | 0 |
| (VI-2) | 0,5 | 1 | 1 | 4 | 1 | 1 | 3 | 1 | 1 | 0 |

**Ansprüche**

1. 2-Pyridyl-oxyacetanilid der Formel

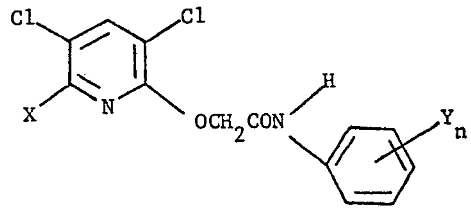

(I)

in der

X Wasserstoff, $C_1$-$C_4$-Alkyl oder $C_1$-$C_4$-Halogenoalkyl bezeichnet,

Y jeweils unabhängig Halogen, $C_1$-$C_4$-Alkyl, $C_1$-$C_4$-Alkoxy oder $C_1$-$C_4$-Halogenoalkyl bezeichnet und

n 0, 1, 2 oder 3 ist.

2. Verbindung nach Anspruch 1, dadurch gekennzeichnet, daß

X Wasserstoff, Methyl, Mono-, Di- oder Trichloromethyl oder Mono-, Di- oder Trifluoromethyl bezeichnet,

Y jeweils unabhängig Fluor, Chlor, Brom, Methyl, Ethyl, n-Propyl, iso-Propyl, Methoxy, Ethoxy, n-Propoxy, iso-Propoxy, Mono-, Di- oder Trichloromethyl oder Mono-, Di- oder Trifluoromethyl bezeichnet und

n 0, 1 oder 2 ist.

3. 3,5-Dichloro-2-pyridyloxyacetanilid der Formel

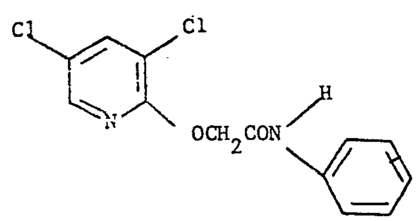

4. 3,5-Dichloro-6-methyl-2-pyridyloxyacetanilid der Formel

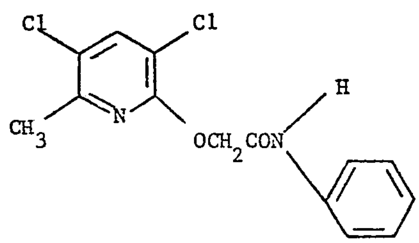

5. Verfahren zur Herstellung einer Verbindung nach Anspruch 1, dadurch gekennzeichnet, daß eine Verbindung der allgemeinen Formel

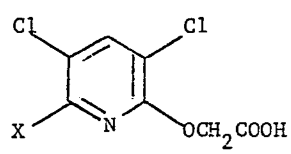

(II)

in der X die in Anspruch 1 angegebene Bedeutung hat, und eine Verbindung der allgemeinen Formel

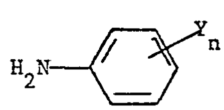

(III)

in der Y und n die in Anspruch 1 angegebenen Bedeutungen haben, unter Wasserabspaltung, gegebenenfalls in einem Lösungsmittel oder Verdünnungsmittel und/oder in Gegenwart eines Kondensationsmittels, kondensiert werden.

6. Herbizide Mittel, gekennzeichnet durch einen Gehalt an mindestens einem 2-Pyridyl-oxyacetanilid der Formel (I) gemäß Anspruch 1.

7. Verwendung von 2-Pyridyl-oxyacetaniliden der Formel (I) gemäß Anspruch 1 zur Bekämpfung von Unkraut.

8. Verfahren zur Herstellung von herbiziden Mitteln, dadurch gekennzeichnet, daß man 2-Pyridyl-oxyacetanilide der Formel (I) gemäß Anspruch 1 mit Streckmitteln und/oder oberflächenaktiven Mitteln vermischt.

**Claims**

1. 2-Pyridyloxyacetanilide of the formula

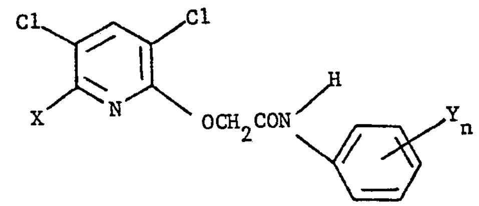

(I)

in which

X designates hydrogen, $C_1$-$C_4$-alkyl or $C_1$-$C_4$-halogenoalkyl,

Y each independently designates halogen, $C_1$-$C_4$-alkyl, $C_1$-$C_4$-alkoxy or $C_1$-$C_4$-halogenoalkyl and

n is 0, 1, 2 or 3.

2. Compound according to Claim 1, characterised in that

X designates hydrogen, methyl, mono-, di- or trichloromethyl or mono-, di- or trifluoromethyl,

Y each independently designates fluorine, chlorine, bromine, methyl, ethyl, n-propyl, isopropyl, methoxy, ethoxy, n-propoxy, iso-propoxy, mono-, di- or trichloromethyl or mono-, di- or trifluoromethyl and

n is 0, 1 or 2.

3. 3,5-Dichloro-2-pyridyloxyacetanilide of the formula

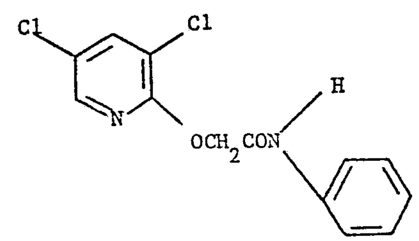

4. 3,5-Dichloro-6-methyl-2-pyridyloxyacetanilide of the formula

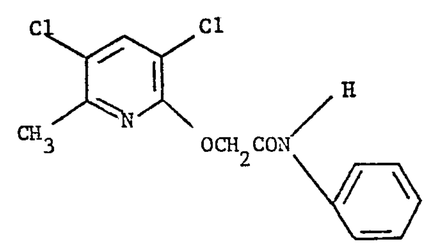

5. Process for the preparation of a compound according to Claim 1, characterised in that a compound of the general formula

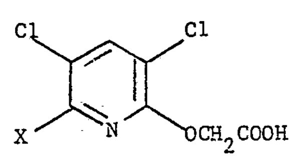

(II)

in which X has the meaning indicated in Claim 1, and a compound of the general formula

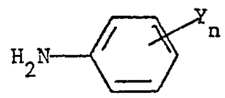

(III)

in which Y and n have the meanings indicated in Claim 1, are condensed, with the elimination of water, if appropriate in a solvent or diluent and/or in the presence of a condensing agent.

6. Herbicidal agent, characterised in that it contains at least one 2-pyridyloxyacetanilide of the formula (I) according to Claim 1.

7. Use of 2-pyridyloxyacetanilides of the formula (I) according to Claim 1 for combating weeds.

8. Process for the preparation of herbicidal agents, characterised in that 2-pyridyloxyacetanilides of the formula (I) according to Claim 1 are mixed with extenders and/or surface-active agents.

**Revendications**

1. 2-Pyridyl-oxyacétanilide de formule

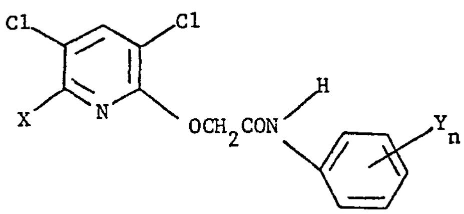

(I)

dans laquelle

X représente l'hydrogène ou un groupe alkyle en $C_1$-$C_4$ ou halogénoalkyle en $C_1$-$C_4$,

Y représente chaque fois indépendamment un halogène ou un groupe alkyle en $C_1$-$C_4$, alcoxy en $C_1$-$C_4$ ou halogénoalkyle en $C_1$-$C_4$ et

n est égal à 0, 1, 2 ou 3.

2. Composé selon la revendication 1, caractérisé en ce que

X représente l'hydrogène ou un groupe méthyle, mono-, di- ou trichlorométhyle ou mono-, di- ou trifluorométhyle,

Y représente chaque fois indépendamment le fluor, le chlore, le brome ou un groupe méthyle, éthyle, n-propyle, isopropyle, méthoxy, éthoxy, n-propoxy, isopropoxy, mono-, di- ou trichlorométhyle, ou mono-, di- ou trifluorométhyle et

n est égal à 0, 1 ou 2.

3. 3,5-Dichloro-2-pyridyloxyacétanilide de formule

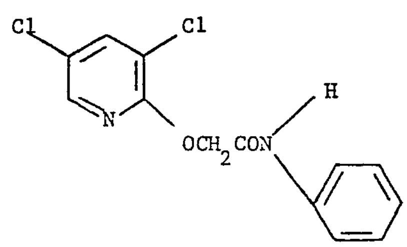

4. 3,5-dichloro-6-méthyl-2-pyridyloxyacétanilide de formule

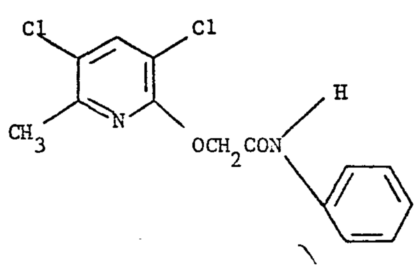

5. Procédé pour la fabrication d'un composé selon la revendication 1, caractérisé en ce que l'on condense un composé de formule générale

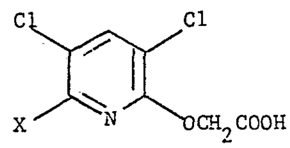

(II)

dans laquelle X a la signification indiquée dans la revendication 1, et un composé de formule générale

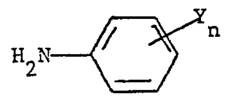

(III)

dans laquelle Y et n ont les significations indiquées dans la revendication 1, avec élimination d'eau, éventuellement dans un solvant ou diluant et/ou en présence d'un agent de condensation.

6. Agents herbicides, caractérisés en ce qu'ils contiennent au moins un 2-pyridyl-oxyacétanilide de formule (I) selon la revendication 1.

7. Utilisation de 2-pyridyl-oxyacétanilides de formule (I) selon la revendication 1 pour la lutte contre les mauvaises herbes.

8. Procédé pour la fabrication d'agents herbicides, caractérisé en ce que l'on mélange des 2-pyridyl-oxyacétanilides de formule (I) selon la revendication 1 avec des agents diluants et/ou des agents tensioactifs.